# EUROPÄISCHE PATENTANMELDUNG

(11) **EP 3 766 358 A1**
(43) Veröffentlichungstag der Anmeldung: **20.01.2021**
(21) Anmeldenummer: 20186763.7
(22) Anmeldetag: 20.07.2020
(51) Int. Cl.: A23K 10/26, A23K 50/10, A61K 35/57

(54) **VERWENDUNG EINES ERGÄNZUNGSFUTTERMITTELS ZUR VERMEIDUNG VON DURCH CALCIUMMANGEL BEDINGTEN STOFFWECHSELSTÖRUNGEN SOWIE ERGÄNZUNGSFUTTERMITTEL**

(30) Priorität: 19.07.2019 DE 102019119651
(71) Anmelder: Gülker, Ludwig, 28857 Ristedt (DE)
(72) Erfinder: Gülker, Ludwig, 28857 Ristedt (DE)
(74) Vertreter: Jabbusch, Matthias

(57) **Zusammenfassung**

Die Erfindung betrifft ein Ergänzungsfuttermittel zur Vermeidung von durch Calciummangel bedingten Stoffwechselstörungen mit wenigstens einem Calciumträger, der aus Eischalen samt ihrer Eihäute besteht. Außerdem betrifft die Erfindung eine Verwendung eines Ergänzungsfuttermittels zur Vermeidung von durch Calciummangel bedingten Stoffwechselstörungen, wobei das Ergänzungsfuttermittel wenigstens einen Calciumträger aufweist, der aus Eischalen samt ihrer Eihäute besteht.

## Beschreibung

Die Erfindung betrifft ein Ergänzungsfuttermittel zur Vermeidung von durch Calciummangel bedingten Stoffwechselstörungen mit wenigstens einem Calciumträger, der aus Eischalen samt ihrer Eihäute besteht. Außerdem betrifft die Erfindung eine Verwendung eines Ergänzungsfuttermittels zur Vermeidung von durch Calciummangel bedingten Stoffwechselstörungen, wobei das Ergänzungsfuttermittel wenigstens einen Calciumträger aufweist, der aus Eischalen samt ihrer Eihäute besteht.

Der für Ergänzungsfuttermittel wohl gebräuchlichste Calciumträger ist Futterkalk, ein aus ungebranntem Kalkstein zermahlenes Pulverpräparat, welches Futtermitteln bedarfsweise beigemengt wird. Ein anderer bekannter Calciumträger ist Muschelkalk, auch Muschelgrit genannt, ein aus zermahlenen Muschelschalen hergestelltes Ergänzungsfuttermittel, welches insbesondere aus der Geflügelhaltung bekannt ist. Ein weniger häufig in Ergänzungsfuttermitteln verwerteter Calciumträger sind bei der Lebensmittelproduktion anfallende Eierschalenabfälle. Diese Verwertungsmöglichkeit ist Gegenstand wissenschaftlicher Publikationen, beispielsweise in "Application of eggshell wastes as valuable and utilizable products: A review" von H. Faridi und Akbar Arabhousseini in Res. Agr. Eng. Vol. 64, 2018 (2): S. 104-114 oder in "Effects of eggshell calcium on productive performance, plasma calcium, bone mineralization and gonadal characteristics in laying hens" von N. Gongruttananum in Poultry Sience 90: 2011, S. 524-529. Von wirtschaftlich besonderer Bedeutung ist die Vermeidung des bei Milchvieh, insbesondere bei Milchkühen, auftretenden Milchfiebers, einem nach der Geburt, insbesondere dem Abkalben, entstehenden Calciummangel, der unbehandelt häufig zum Verlust des betroffenen Muttertieres führt. Zur Prävention von Milchfieber hat sich eine bloße Verfütterung von Futterkalk und Muschelkalk als nicht ausreichend gezeigt, eine bloße Verfütterung der in der Geflügelhaltung diskutierten Eierschalenabfälle wurde nicht versucht, da bereits seit langem bekannt war, dass eine präventive Wirkung nur mit ganzheitlichen Fütterungskonzepten erreicht werden konnte, wie beispielsweise die sogenannte Transitfütterung mit einem mehrstufigen Fütterungsplan vor der Geburt, die orale, subkutane oder intravenöse Verabreichung von Calciumpräparaten unmittelbar nach der Geburt, oder komplexe Fütterungskonzepte unter Ausnutzung der Kationen-Anionen-Bilanz in den Futtermitteln. Eine erfolgreiche Anwendung dieser Konzepte erfordert jedoch einschlägige Kenntnisse über die biochemischen Abläufe bei der Calciumversorgung der Tiere und in den landwirtschaftlichen Betrieben ein entsprechend geschultes Personal, welches die Konzepte erfolgreich umzusetzen vermag.

Der Erfindung liegt deshalb die Aufgabe zugrunde, ein Ergänzungsfuttermittel aufzuzeigen, mit welchem durch Calciummangel bedingtes Milchfieber besonders einfach und gleichwohl wirksam vermeidbar ist.

Diese Aufgabe ist erfindungsgemäß mit einem Ergänzungsfuttermittel gemäß den Merkmalen des Patentanspruches 1 gelöst. Vorteilhafte Weiterbildungen der Erfindung sind in den Unteransprüchen angegeben.

Das erfindungsgemäße Ergänzungsfuttermittel zeichnet sich demnach dadurch aus, dass das Ergänzungsfuttermittel für die Verfütterung an Milchvieh, insbesondere an Milchkühe, konditioniert ist. Die erfindungsgemäße Verwendung eines Ergänzungsfuttermittels, welches einen Calciumträger aus Eischalen samt ihrer Eihäute aufweist, zeichnet sich dadurch aus, dass das Ergänzungsfuttermittel zur Vermeidung und zur Behandlung von Milchfieber an Milchvieh, insbesondere an Milchkühe, verfüttert wird. Es wurde überraschend festgestellt, dass die Eischalen samt ihrer Eihäute eine hinreichende Calciumversorgung der Muskulatur und des Nervensystems des Milchviehs gewährleisten. Insbesondere in den Eihäuten enthaltenes Vitamin D₃ verbessert dabei den Calciumtransport im Blutkreislauf der Nutztiere und ebenfalls maßgeblich in den Eihäuten enthaltenes Vitamin K₂ wird für die Ein- und Auslagerung des Calciums in das Skelett hinein bzw. aus diesem heraus benötigt. Somit stellt das in der Legehennenhaltung anfallende Restprodukt aus Eischalen und deren Eihäuten ein Ergänzungsfuttermittel dar, welches durch bloße Beimengung in das Hauptfutter des Milchviehs die bekannten komplexeren Fütterungskonzepte in einer besonders effektiven Weise ersetzen kann.

Nach einer ersten Weiterbildung der Erfindung sind die Eischalen samt ihrer Eihäute ein Restprodukt von Eiern aus Boden- oder Freilandhaltung. Versuche haben ergeben, dass Eischalen und Eihäute aus Boden- oder Freilandhaltung eine höhere Wirksamkeit gegen das Auftreten von durch Calciummangel bedingten Stoffwechselstörungen haben. Die Gründe hierfür sind, dass es den Eischalen oder Eihäuten aus Käfighaltung an Rezeptoren oder Botenstoffe aus der Gruppe der Hormone, Proteine oder Enzyme mangelt, die für ein wirksames Zusammenspiel der Vitamine D₃ und K₂ verantwortlich sind.

Nach einer nächsten Weiterbildung der Erfindung wird deshalb vorgeschlagen, dass der Calciumträger mit wenigstens einem Botenstoff zur Optimierung der Calciumversorgung angereichert ist. Eine solche Anreicherung mit insbesondere Vitamin D3 und K2 würde vorteilhaft die Herstellung des erfindungsgemäßen Ergänzungsfuttermittels aus Eischalen und Eihäuten ermöglichen, die aus der Käfighaltung bzw. Legebatteriehaltung kommen.

Um die Verfügbarkeit des in den Eischalen enthaltenen Calciums sowie der in den Eihäuten enthaltenen Vitamine für das Milchvieh zu verbessern, sind die Eischalen samt ihrer Eihäute zu einem Futterergänzungsmehl zerkleinert. Das Futterergänzungsmehl ausbildende Mehlpartikel weisen eine Partikelgröße von unter 0,5 mm, vorzugsweise von unter 0,2 µm, auf, damit eine hinreichende Verfügbarkeit des Futterergänzungsmehls über die Verstoffwechselung des Tieres möglich ist. Eine vorherige Sterilisation oder Hygienisierung der zu dem erfindungsgemäßen Ergänzungsfuttermittel zu verarbeitenden Eischalen und Eihäute ist unbedingt zu empfehlen und liegt selbstverständlich auch im Rahmen der Erfindung.

Nach einer anderen Weiterbildung der Erfindung wird für eine Fütterung des Milchviehs mit einem Kraftfutter vorgeschlagen, dass das Futterergänzungsmehl in Kraftfutterpresslinge eingebunden ist. Durch eine Verfütterung der mit dem erfindungsgemäßen Ergänzungsfuttermittel hergestellten Kraftfutterpresslinge läßt sich der vom Milchviehhalter zu betreibende Mehraufwand zur Prävention von durch Calciummangel bedingten Stoffwechselstörungen nahezu vollständig eliminieren. Es liegt selbstverständlich im Rahmen der Erfindung, das Futterergänzungmehl in Pellets oder Granulate einzubinden oder totalen Mischrationen TMR, Konzentratfutter, Kraftfutter oder sogar anderen Ergänzungsfuttermitteln unterzumischen. Das Risiko, dass Milchvieh an Milchfieber oder einer anderen durch einen Calciummangel bedingten Stoffwechselstörung erkrankt, ist mit dem erfindungsgemäßen Ergänzungsfuttermittel signifikant herabgesetzt.

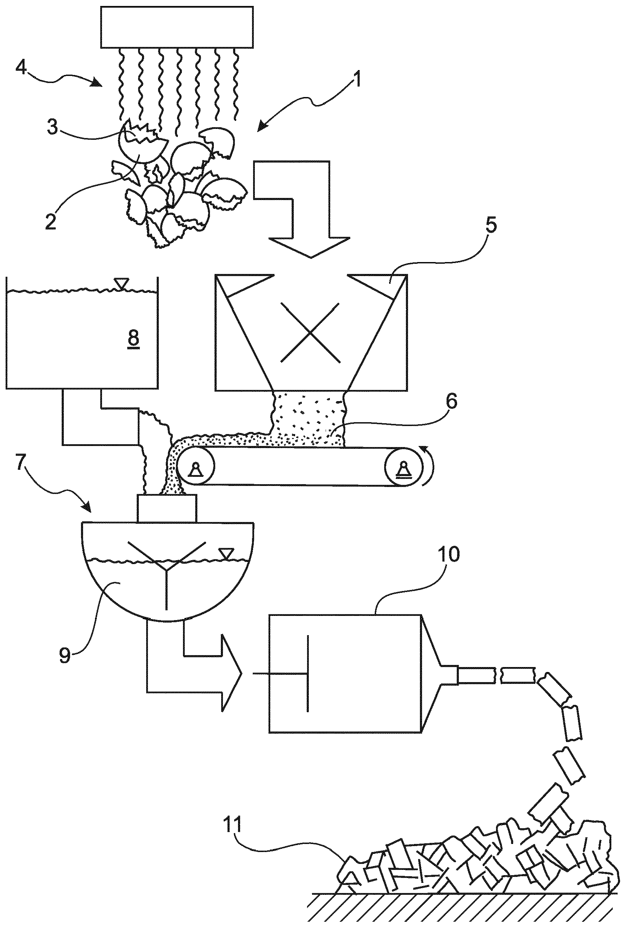

Ein Ausführungsbeispiel der Erfindung, aus dem sich weitere erfinderische Merkmale ergeben, ist in der Zeichnung dargestellt.
Die einzige Figur zeigt eine schematische Darstellung der Herstellung eines erfindungsgemäßen Ergänzungsfuttermittels mit einem Calciumträger 1 aus Eischalen 2 samt ihrer Eihäute 3, die zur Hygienisierung einer Trocknung mittels Warmluft 4 zugeführt werden. Die hygienisierten Eischalen 2 werden samt ihrer Eihäute 3 in einem Mahlwerk 5 zu einem Futterergänzungsmehl 6 zermahlen. Sodann wird das Futterergänzungsmehl 6 in einem Mischwerk 7 mit einem Kraftfutterbrei 8 zu einem Fertigbrei 9 vermengt, der schließlich in einem Futterextruder 10 zu Kraftfutterpresslingen 11 endverarbeitet wird.

## Patentansprüche

1. Verwendung eines Ergänzungsfuttermittels zur Vermeidung von durch Calciummangel bedingten Stoffwechselstörungen, wobei das Ergänzungsfuttermittel wenigstens einen Calciumträger aufweist, der aus Eischalen samt ihrer Eihäute besteht,
**dadurch gekennzeichnet,**
**dass** das Ergänzungsfuttermittel zur Vermeidung und Behandlung von Milchfieber an Milchvieh verfüttert wird.

2. Verwendung eines Ergänzungsfuttermittels nach Anspruch 1, bei dem die Eischalen (2) samt ihrer Eihäute (3) von Eiern aus Boden- oder Freilandhaltung sind.

3. Verwendung eines Ergänzungsfuttermittels nach Anspruch 1 oder 2, bei dem der Calciumträger (1) mit wenigstens einem Botenstoff zur Optimierung der Calciumversorgung angereichert ist.

4. Verwendung eines Ergänzungsfuttermittels nach einem der Ansprüche 1 bis 3, bei dem die Eischalen (2) samt ihrer Eihäute (3) zu einem Futterergänzungsmehl (6) zerkleinert sind.

5. Verwendung eines Ergänzungsfuttermittels nach Anspruch 4, bei dem das Futterergänzungsmehl (6) in Kraftfutterpresslingen (11) eingebunden ist.

6. Ergänzungsfuttermittel zur Vermeidung von durch Calciummangel bedingten Stoffwechselstörungen mit wenigstens einem Calciumträger, der aus Eischalen samt ihrer Eihäute besteht,
**dadurch gekennzeichnet,**
**dass** das Ergänzungsfuttermittel für die Verfütterung an Milchvieh konditioniert ist.

7. Ergänzungsfuttermittel nach Anspruch 6, **dadurch gekennzeichnet, dass** die Eischalen (2) samt ihrer Eihäute (3) von Eiern aus Boden- oder Freilandhaltung sind.

8. Ergänzungsfuttermittel nach Anspruch 6 oder 7, **dadurch gekennzeichnet, dass** der Calciumträger (1) mit wenigstens einem Botenstoff zur Optimierung der Calciumversorgung angereichert ist.

9. Ergänzungsfuttermittel nach einem der Ansprüche 6 bis 8, **dadurch gekennzeichnet, dass** die Eischalen (2) samt ihrer Eihäute (3) zu einem Futterergänzungsmehl (6) zerkleinert sind.

10. Ergänzungsfuttermittel nach Anspruch 9, **dadurch gekennzeichnet, dass** das Futterergänzungsmehl (6) in Kraftfutterpresslingen (11) eingebunden ist.
